# EUROPÄISCHE PATENTANMELDUNG

(11) **EP 0 750 893 A2**
(43) Veröffentlichungstag der Anmeldung: **02.01.1997**
(21) Anmeldenummer: 96107547.0
(22) Anmeldetag: 11.05.1996
(51) Int. Cl.: A61F 13/15

(54) **Verfahren und Vorrichtung zur Herstellung von Windeln od. dgl. mit mindestens einem dehnungselastischen Bereich**

(30) Priorität: 29.05.1995 DE 19518995; 22.06.1995 DE 19522743
(71) Anmelder: Hyga Produktion GmbH & Co. KG, 36124 Eichenzell (DE)
(72) Erfinder: Filip, Michael, Ing., 36088 Hünfeld (DE); Kutsche, Joachim, Ing., 36124 Eichenzell (DE); Breitenbach, Ulrich, Ing., 36124 Eichenzell (DE)
(74) Vertreter: Gesthuysen, von Rohr & Weidener

(57) **Zusammenfassung**

Gegenstand der Erfindung ist ein Verfahren zur Herstellung von Windeln oder dgl. mit mindestens einem dehnungselastischen Bereich, sowie eine entsprechende Herstellungsvorrichtung. Bei diesem Verfahren wird eine Nichtelastiklage (8) mit in Querrichtung (quer zur Längsrichtung) des Dehnungselements (6) verlaufenden Falten bzw. Kräuselungen versehen und in diesem Zustand stabilisiert. Die Nichtelastiklage (8) wird mit einer Elastiklage (7) verbunden. Die in Längsrichtung dehnbare Elastiklage (7) wird dabei nicht gedehnt. Die Herstellungs-Bewegungsrichtung ist die Querrichtung des in Längsrichtung dehnbaren, regelmäßig streifenförmigen Dehnungselementes, das aus der in Längsrichtung dehnbaren Elastiklage (7) und mindestens einer Nichtelastiklage (8) besteht. Damit ist die Durchlaufrichtung des Materials in dieser Herstellungsphase gleich der Herstellungs-Bewegungsrichtung der Windel-Herstellungslinie insgesamt, ein kontinuierlicher Durchlauf in der Windel-Herstellungslinie ist also gewährleistet.

## Beschreibung

Die Erfindung betrifft ein Verfahren zur Herstellung einer Windel od. dgl. mit den Merkmalen des Oberbegriffs von Anspruch 1 und eine entsprechende Herstellungsvorrichtung mit den Merkmalen des Oberbegriffs von Anspruch 11 bzw. 12.

Die Erfindung befaßt sich mit der Herstellung von Windein und ähnlichen Produkten mit mindestens einem dehnungselastischen Bereich. Im Sinne der vorliegenden Erfindung sind Windeln einerseits Babywindeln, andererseits Erwachsenenwindeln, jedoch auch andere Inkontinenzprodukte und entsprechende Textilien. Die Lehre der Erfindung betrifft also nicht nur Windein im engeren Sinne, insbesondere Babywindeln, sondern neben Erwachsenenwindeln beispielsweise auch Slipeinlagen, Damenbinden, Inkontinenzhöschen (training pants), sonstige ähnliche Hygieneprodukte, sowie Textilprodukte, insbesondere entsprechende Höschen, die alle darin übereinstimmen, daß sie mindestens einen dehnungselastischen Bereich, insbesondere im Bundbereich aufweisen. Wenn nachfolgend jeweils von einer "Windel" die Rede ist, so ist generell zu verstehen, daß die oben angeführten gattungsähnlichen Produkte damit ebenso gemeint sind. Die Verwendung des Begriffs "Windel" dient lediglich der einfacheren Erläuterung und berücksichtigt die Tatsache, daß bei Babywindeln und Erwachsenenwindeln ein besonderer Anwendungsbereich der vorliegenden Erfindung zu sehen ist.

Bei bekannten Windein wird der dehnungselastische Bereich dadurch realisiert, daß in diesem Bereich ein in Längsrichtung dehnbares, vorzugsweise streifenförmiges Dehnungselement aus einer in Längsrichtung dehnbaren Elastiklage und mindestens einer damit flächig verbundenen, insbesondere verklebten oder - durch Ultraschallschweißung - verschweißten Nichtelastiklage angeordnet ist, dessen Nichtelastiklage bei nicht gedehnter Elastiklage gefältelt bzw. gekräuselt und bei gedehnter Elastiklage im Grenzzustand gestrafft bzw. glatt ist. Solche Beispiele finden sich beispielsweise in der EP - B - 0 323 040, ein entsprechendes Herstellungsverfahren ist u. a. Gegenstand der US - A - 4,735,673.

Generell ist es so, daß das Dehnungselement nicht nur aus der Zweierkombination Elastiklage/Nichtelastiklage, sondern auch aus einer Dreierkombination Nichtelastiklage/Elastiklage/Nichtelastiklage bestehen kann. Bei einer Zweierkombination Elastiklage/Nichtelastiklage wird dieses Dehnungselement mit der Seite der Elastiklage meist mit dem Basismaterial der Windel in entsprechender Weise verklebt bzw. verschweißt.

Dehnungselemente werden bislang häufig so hergestellt, daß die Elastiklage in gedehntem Zustand mit der Nichtelastiklage vollflächig verbunden, insbesondere verklebt oder verschweißt wird, so daß sich die Fältelung bzw. Kräuselung der Nichtelastiklage von selbst ergibt, wenn die äußere Haltespannung von der Elastiklage bzw. dem Dehnungselement entfernt wird. Die sich zusammenziehende Elastiklage kräuselt bzw. fältelt die Nichtelastiklage.

Man kann die zuvor angesprochene Verfahrenstechnik mit längsdehnbarem Material für die Elastiklage realisieren, dann wird das kontinuierlich durchlaufende Material der Elastiklage in Längsrichtung gedehnt (durch zwei hintereinander angeordnete, mit unterschiedlicher Umlaufgeschwindigkeit laufende Dehnungswalzen einer Dehnungsstation). Hierbei besteht eine Schwierigkeit darin, daß die Herstellungs-Bewegungsrichtung - Richtung der Längsdehnung der Elastiklage - bezüglich der Herstellungs-Bewegungsrichtung der Windel-Herstellungslinie selbst quer verläuft. Die Aufbringung des in Längsrichtung gedehnten Dehnungselementes auf das Basismaterial der Windel erfordert einen 90° Übergabevorgang, der bei schnell durchlaufenden Herstellungsverfahren schwierig zu realisieren und jedenfalls maschinell und steuerungstechnisch aufwendig ist.

Das aus der US - A - 4,735,673 bekannte Verfahren arbeitet demgegenüber mit querdehnbarem Material für die Elastiklage, so daß die Dehnungsrichtung der Elastiklage gleich der Querrichtung der Windel-Herstellungslinie ist. Hier entfällt die 90°-Übergabe, man muß den Dehnungsvorgang jedoch quer zur Herstellungs-Bewegungsrichtung der Elastiklage selbst realisieren. Das hat zur Folge, daß man an den Längsrändern des zunächst kontinuierlich durchlaufenden Materialstreifens für die Elastiklage mit Greifern angreifen und das Material in Querrichtung dehnen und in dieser gedehnten Lage festhalten und mit der Nichtelastiklage verbinden muß. Das erfordert zusätzliches Material nur zum Realisieren der Angriffsflächen für die Quergreifer. Etwa 25 bis 30 % bezogen auf die Länge des Dehnungselementes sind überflüssige Randstreifen. Das schlägt sich kostenmäßig bei der Herstellung ganz erheblich nieder, wenn man berücksichtigt, daß etwa 80 % der Kosten die Herstellung solcher Dehnungselemente, die wiederum einen beachtlichen Kostenfaktor für die Herstellung insgesamt ausmachen, in den Materialkosten liegt. Ein weiteres Problem bei Einsatz querdehnbaren Materials für die Elastiklage besteht darin, daß das moderne, im Gegensatz zu PU-Schaum sehr hautfreundliche "fluted elastic material" so dünn und reißgefährdet ist, daß man es im Querdehnungsverfahren kaum einsetzen kann.

Die voranstehend erläuterten Schwierigkeiten sind bereits erkannt und einer weiter verbesserten Lösung zugeführt worden, bei der die Vorteile der Herstellung mit längsdehnbarem Material in etwa mit den Vorteilen der Herstellung mit querdehnbarem Material zusammenfallen. Die grundlegende Idee für diesen Stand der Technik, von dem die Erfindung letztlich ausgeht, besteht darin, die Elastiklage während der Herstellung nicht in ihrer Längsrichtung zu dehnen, sondern ungedehnt mit der Nichtelastiklage zu verbinden, jedoch die Nichtelastiklage demgegenüber vor dem Verbinden mit der Elastiklage zu fälteln bzw. zu kräuseln (DE - A - 30 16 197, EP - A - 0 182 942).

Bei dem zuvor erläuterten Stand der Technik fällt die Herstellungs-Bewegungsrichtung mit der Längsrichtung des Dehnungselements zusammen. In der jeweiligen Herstellungsvorrichtung verlaufen entsprechende Nuten auf einer Fältelungswalze auf deren Mantel in Achsrichtung.

Die voranstehenden Ausführungen machen deutlich, daß bei allen Vorteilen des zuvor erläuterten, den Ausgangspunkt für die Lehre bildenden Standes der Technik hier doch wieder das Problem auftritt, daß zur Einführung in die Windel-Herstellungslinie ein 90°-Übergabevorgang für das Dehnungselement erforderlich ist. Das gleichzeitige Erreichen der Vorteile der Herstellung mit längdehnbarem und querdehnbarem Material gelingt also mit dem zuvor erläuterten Verfahren nicht vollständig.

Der Erfindung liegt die Aufgabe zugrunde, das zuvor erläuterte Herstellungsverfahren so auszugestalten und weiterzubilden, daß wirklich alle Vorteile der Herstellung mit längsdehnbarem Material und der Herstellung mit querdehnbarem Material zugleich erreicht werden, wobei dieses Verfahren möglichst kostengünstig sein soll. Gegenstand der Erfindung ist auch die Angabe einer entsprechenden Herstellungsvorrichtung.

Die zuvor aufgezeigte Aufgabe ist bei einem Verfahren mit den Merkmalen des Oberbegriffs von Anspruch 1 durch die Merkmale des kennzeichnenden Teils von Anspruch 1 gelöst. Mit der erfindungsgemäß getroffenen Maßnahme ist die Längsrichtung des Dehnungselementes quer zur Herstellungs-Bewegungsrichtung ausgerichtet, also auch quer bezüglich des kontinuierlichen Durchlaufs in der Windel-Herstellungslinie. Damit kann das Dehnungselement ohne 90°-Übergabevorgang in die Windel-Herstellungslinie einlaufen.

Bevorzugte Ausgestaltungen und Weiterbildungen des erfindungsgemäßen Verfahrens sind Gegenstand der Ansprüche 2 bis 10. Die Ansprüche 11 und 12 beschreiben jeweils eine vorteilhafte Herstellungsvorrichtung.

Im folgenden wird die Erfindung anhand einer lediglich Ausführungsbeispiele darstellenden Zeichnung näher erläutert. In der Zeichnung zeigt
- Fig. 1: in perspektivischer Darstellung eine heutzutage übliche Babywindel, stark schematisiert,
- Fig. 2: eine perspektivische Darstellung eines Teilbereichs einer Herstellungsvorrichtung für eine erfindungsgemäße Windel,
- Fig. 3: den in Fig. 2 dargestellten Teilbereich in einer schematischen Seitenansicht und
- Fig. 4: einen anders konstruierten Teilbereich einer Herstellungsvorrichtung für eine erfindungsgemäße Windel.

Wie schon eingangs erläutert worden ist, steht der Begriff Windel hier für eine Vielzahl von Zellstoff-/Kunststoff-Produkten mit mindestens einem dehnungselastischen Bereich, vorzugsweise angeordnet im Bundbereich, also beispielsweise für Slipeinlagen und Damenbinden sowie Textilprodukte.

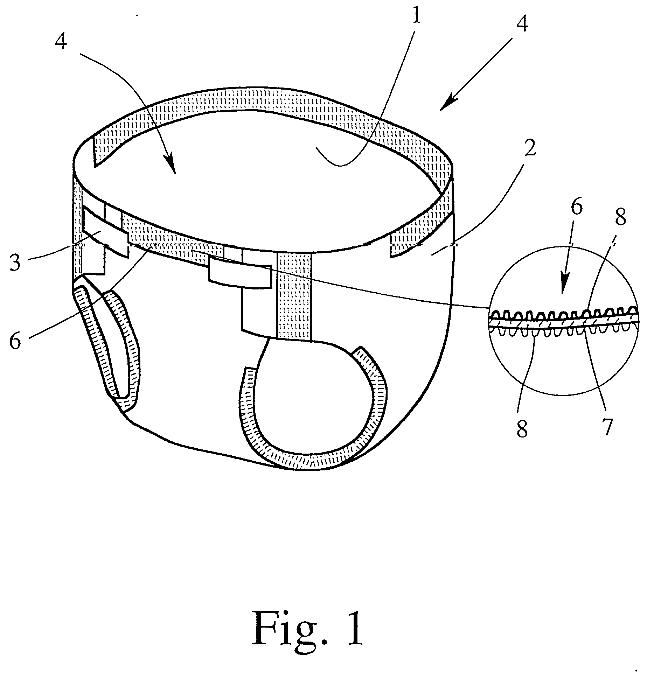

Die in Fig. 1 schematisch dargestellte Windel besteht zunächst aus einer oberen, beim Anlegen zur Haut orientierten, flüssigkeitsdurchlässigen Oberlage 1, (Fachjargon: Topsheet), einem flüssigkeitsaufnehmenden Kern, der entweder aus defibriertem Zellstoff allein oder aus einem Gemisch von zerfasertem Zellstoff und Superabsorberstoff besteht (Core), sowie einer beim Anlegen zur Außenseite orientierten, flüssigkeitsundurchlässigen Unterlage 2 (Backsheet), die gemeinhin als PE-Folie ausgeführt ist.

Unter Umständen befinden sich auf der zur Haut orientierten Seite des Kerns noch andere Lagen, die der besseren Verteilung der Flüssigkeit dienen (Distribution Lavers). Auf der nach außen orientierten Seite des Kerns können noch weitere Lagen flüssigkeitsaufnehmend und speichernd wirken (Aquisition Layers).

Auf der inneren oder äußeren Seite der Unterlage 2 wird im Bereich des vorderen Teils ein Streifen Folie aufgetragen (Frontal Tape), der die Festigkeit der Unterlage 2 in diesem Bereich erhöht, so daß die Möglichkeit besteht, die Windel mit zwei seitlichen Klebestreifen 3 (Sidetapes) zu schließen.

Die weitere Ausgestaltung von Windeln mit dem Abschlußstreifen (Waist Shield), mit im Inneren aufstehenden Abdichtstreifen (Standing Cuffs) und elastischen Beinabschlüssen bedarf hier keiner weiteren Erläuterung, da das alles Stand der Technik ist.

Interessant ist der dehnungselastische Bereich 4 (mindestens ein solcher Bereich ist vorgesehen), der vorzugsweise im Bundbereich angeordnet ist. Dieser dehnungselastische Bereich 4 befindet sich im in Fig. 1 dargestellten Beispiel einer Windel nicht nur im Bundbereich vorn, sondern auch hinten. Hier sind sogar weitere dehnungselastische Bereiche 4 in den Seitenlaschen der Windel vorgesehen (Sideflaps). Diese dehnungselastischen Bereiche 4 ersetzen die zuvor erläuterten Flüssigkeitssperren (Waist Shield) und übernehmen die Rolle der Flüssigkeitssperren und der Rumpfanpassung und die Sicherung gegen ein Verrutschen der Windel nach unten auf dem Körper des Trägers.

In Fig. 1 herausgezeichnet ist das Element, mit dem ein solcher dehnungselastischer Bereich 4 in einer Windel ausgebildet wird. Das geschieht nämlich durch ein in Längsrichtung dehnbares, hier und üblicherweise streifenförmiges Dehnungselement 6 aus einer in Längsrichtung dehnbaren Elastiklage 7 und mindestens einer damit flächig verbundenen, insbesondere verklebten - oder durch Ultraschallschweißung - verschweißten Nichtelastiklage 8. Für die Elastiklage 7 kommt insbesondere das Material "fluted elastic material" in Frage, das besonders gesundheitsfreundlich ist, während für die Nichtelastiklage 8 normale PE-Folie eingesetzt werden kann. Die in Fig. 1 herausgezeichnete Darstellung zeigt im übrigen eine weitere Nichtelastiklage 8 auf der anderen Seite der Elastiklage 7, also ein dreilagiges Dehnungselement 6.

Fig. 1 im herausgezeichneten Bereich zeigt weiter, daß die Nichtelastiklage 8 bei nicht gedehnter Elastiklage 7, wie in Fig. 1 herausgezeichnet dargestellt, gefältelt bzw. gekräuselt und, so in Fig. 1 nicht dargestellt, bei gedehnter Elastiklage 7 gestrafft bzw. glatt ist. Dadurch erreicht man den gewünschten elastischen Effekt im Bereich 4.

Es ist bekannt, Windeln der in Rede stehenden Art mit dem zuvor beschriebenen, bekannten Dehnungselement 6 so auszugestalten, daß dieses auf der Unterlage 2 auf der Außenseite angebracht, insbesondere dort verklebt oder verschweißt ist, oder auf der inneren Seite der Oberlage 1 oder auch zwischen Oberlage 1 und Unterlage 2 dazwischen. Die in Fig. 1 im herausgezeichneten Bereich punktiert gezeichnete untere Nichtelastiklage 8 kann auch durch das Folienmaterial der Unterlage 2 der Windel selbst gebildet werden, wobei das Dehnungselement 6 jedenfalls in gedehntem Zustand mit dem flächigen Material der Oberlage 1 und/oder der Unterlage 2 verbunden werden muß, so daß das Material der Oberlage 1 bzw. Unterlage 2 beim Zusammenziehen der Elastiklage 7 mit eingezogen wird.

Eine Alternative besteht allerdings auch darin, Dehnungsschlitze in Oberlage 1 und/oder Unterlage 2 anzubringen, so daß bei Dehnung des Dehnungselements 6 die Oberlage 1 bzw. die Unterlage 2 der Dehnung folgen können.

Übliche Dehnungselemente 6 erlauben eine Dehnung um 20 % bis 100 %, üblich sind meistens 30 % bis 50 % Dehnung (Länge des gedehnten Dehnungselements 6 verglichen mit der Länge des nicht gedehnten Dehnungselements 6).

Fig. 1 zeigt im herausgezeichneten Bereich 4, daß die gefältelte bzw. gekräuselte Nichtelastiklage 8 in Längsrichtung gesehen nur streifenweise bzw. punktweise mit dazwischen befindlichen nicht verbundenen Abschnitten mit der Elastiklage 7 verbunden ist.

Das beschriebene Verfahren arbeitet so, daß die Nichtelastiklage 8 mit in Querrichtung (quer zur Längsrichtung des Dehnungselements 6) verlaufenden Falten bzw. Kräuselungen versehen und in diesem Zustand stabilisiert wird, daß die gefältelte bzw. gekräuselte Nichtelastiklage 8 mit der Elastiklage 7 verbunden, insbesondere verklebt oder verschweißt wird, daß die in Längsrichtung dehnbare Elastiklage 7 bei dem zuvor genannten Verfahrensschritt nicht gedehnt ist und daß das so hergestellte Dehnungselement 6 den weiteren Herstellungsschritten zugeführt wird. Wesentlich ist dabei, daß die Herstellungs-Bewegungsrichtung die Querrichtung bezüglich des Dehnungselements 6 ist. Somit ist die Längsrichtung des Dehnungselements 6 richtigerweise quer zur Herstellungs-Bewegungsrichtung ausgerichtet, wie das für den kontinuierlichen Durchlauf in der Windel-Herstellungslinie gewünscht ist.

Das erfindungsgemäße Verfahren bietet die Vorteile der Verarbeitung längsdehnbaren Materials und querdehnbaren Materials ohne die Nachteile dieser Verfahren. Vorteilhaft ist, daß das erfindungsgemäße Verfahren im Längsdurchlauf gefahren werden kann, weil die Durchlaufrichtung des Materials der Nichtelastiklage 8 gleich der Herstellungs-Bewegungsrichtung der Windel-Herstellungslinie insgesamt ist. Das Material der Elastiklage 7, das bezüglich der Querdehnung ja die eigentlichen Probleme aufgeworfen hat, wird erfindungsgemäß zwar bezüglich der Laufrichtung so wie querdehnbares Material verarbeitet, jedoch ohne tatsächliche Querdehnung und damit ohne die Gefahr eines Reißens und ohne die Notwendigkeit, seitliche zusätzliche Randstreifen als Angriffsbereiche für Querdehnungsgreifer zu realisieren. Damit ist der Materialeinsatz für die - teure - Elastiklage 7 erheblich geringer als bei Verarbeitung querdehnbaren Materials in klassischer Weise.

Für das Herstellungsverfahren der Windel od. dgl. gilt im übrigen, daß das Dehnungselement 6 anschließend in Längsrichtung gedehnt und dann dem nächsten Herstellungsschritt zugeführt wird. Damit wird dann das quer zur Herstellungs-Bewegungsrichtung der Windel-Herstellungslinie gedehnte Dehnungselement 6 mit dem Material der Oberlage 1 oder Unterlage 2 der Windel auf der Windel-Herstellungslinie unproblematisch verbunden. Alternativ ist es auch möglich, daß das Dehnungselement 6 nicht in Längsrichtung gedehnt dem nächsten Herstellungsschritt zugeführt wird. Wie im einzelnen mit dem Dehnungselement 6 im weiteren Herstellungsprozeß verfahren wird, ist jedoch nicht mehr Gegenstand der vorliegenden Erfindung.

Das Verfahren kann man so realisieren, daß die Nichtelastiklage 8 während des Verbindens mit der Elastiklage 7 in der Herstellungs-Bewegungsrichtung kontinuierlich bewegt wird.

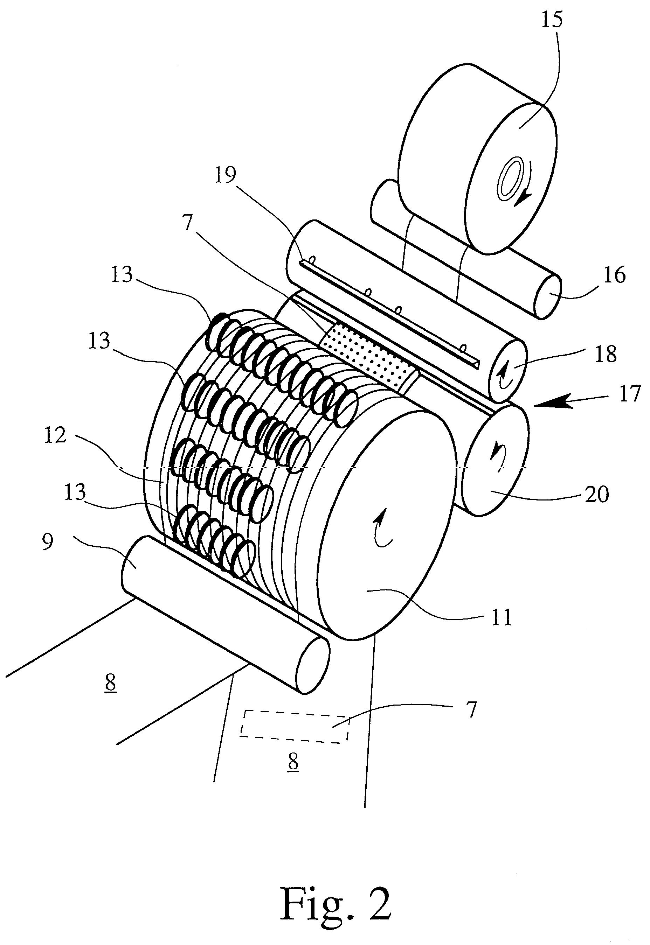

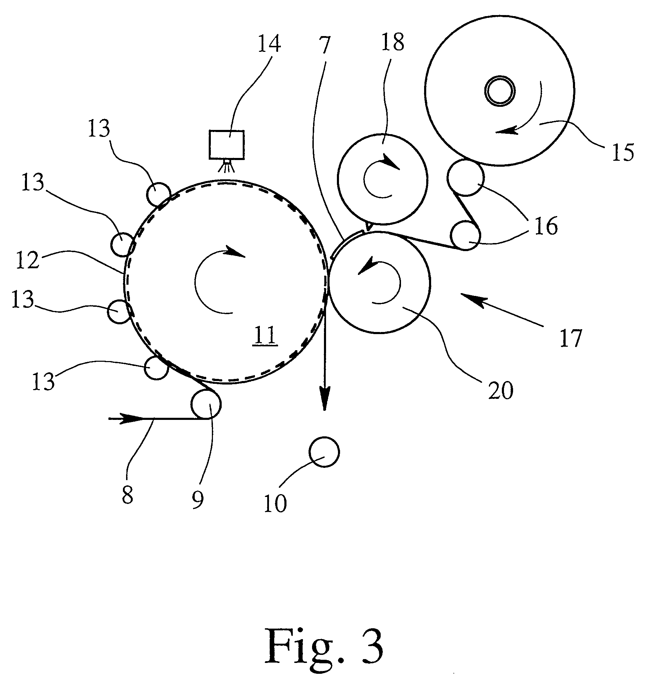

Im einzelnen zeigen Fig. 2 und 3, daß die glatte Nichtelastiklage 8 von einer Vorratswalze durch eine Fältelungsstation zu einer Verbindungsstation mit der Elastiklage 7 gezogen und in der Fältelungsstation von innen nach außen fortschreitend mit den in Bewegungsrichtung verlaufenden Falten versehen wird.

Die Fältelungsstation kann eine lineare Station sein. Einfacher ist die Konstruktion, die in den Fig. 2 und 3 dargestellt ist. Hier wird das kontinuierlich einlaufende Material der Nichtelastiklage 8 (Pfeil) zunächst um eine Umlenkwalze 9 umgelenkt und dann unter Zug, der von einer Abzugswalze 10 aufrechterhalten wird, um eine Fältelungswalze 11 geführt, die über ihre Breite verteilt eine Mehrzahl von Ringnuten 12 aufweist, in die das Material der Nichtelastiklage 8 von innen nach außen fortschreitend durch entsprechend eingreifende Druckrollen 13 faltenartig hineingedrückt wird.

Das in den Fig. 2 und 3 dargestellte Ausführungsbeispiel zeigt insoweit eine bevorzugte Ausführungsform, für die gilt, daß die Nichtelastiklage 8 auf der Fältelungswalze 11 vier in Umfangsrichtung hintereinander angeordnete Gruppen von Druckrollen 13 und danach eine Klebemittel-Auftragstation 14 durchläuft und ungefähr beim Verlassen der Fältelungswalze 11, also beim tangentialen Ablaufen von der Fältelungswalze 11, mit der nicht gedehnten Elastiklage 7 verbunden wird. Anstelle eines Durchlaufens der Klebemittel-Auftragstation 14 kann die Nichtelastiklage 8 auch beim Verbinden mit der Elastiklage 7 - durch Ultraschallschweißung - verschweißt werden. Man erkennt in Fig. 2 vier Gruppen von Druckrollen 13, deren jede in die zugeordnete Ringnut 12 eingreift. Dabei ist hier wesentlich, daß die erste Gruppe von Druckrollen 13 nur mit den in der Mitte der Fältelungswalze 11 befindlichen Ringnuten 12 in Eingriff steht, während die folgenden Gruppen von Druckrollen 13 jeweils weitere äußere Ringnuten 12 erfassen. Dabei sind die folgenden Gruppen von Druckrollen 13 auch immer in den schon in den vorherigen Gruppen von Druckrollen 13 erfaßten Ringnuten 12 aktiv.

Man erkennt im in Fig. 2 dargestellten Ausführungsbeispiel, daß zunächst in der ersten Gruppe sechs Druckrollen 13 angeordnet sind, daß dann in der zweiten Gruppe acht Druckrollen 13 angeordnet sind, während in der dritten Gruppe zehn Druckrollen 13 und in der vierten Gruppe zwölf Druckrollen 13 angeordnet sind. Man erkennt ferner, wie das durchlaufende Material der Nichtelastiklage 8 randseitig von diesen Druckrollen 13 von außen nach innen fortschreitend eingezogen wird. Der durch die Abzugswalze 10 im durchlaufenden Material der Nichtelastiklage 8 aufrechterhaltene Zug gewährleistet, daß das Material der Nichtelastiklage 8 in den Ringnuten 12, die am Nutgrund einen geringeren Durchmesser der Fältelungswalze 11 realisieren, gehalten wird.

Fig. 3 zeigt, daß in der Klebemittel-Auftragstation 14 dann Klebemittel aufgetragen wird, das sich auf dem weiteren Umfang der Fältelungswalze 11 auf der Oberfläche der Nichtelastiklage 8 stabilisiert und dann in der Verbindungsstation zur Verbindung mit dem Material der Elastiklage 7 führt.

Für die Elastiklage 7 gilt, daß diese von einer Vorratswalze 15 kontinuierlich abgezogen und über zwei Umlenk- und Kalanderwalzen 16 einer Schneide- und Distanzstation 17 (Cut and Slip Station) zugeführt wird. Die Schneide- und Distanzstation 17 weist im dargestellten Ausführungsbeispiel eine Schneidewalze 18 mit Schneidemesser 19 sowie eine Vakuumwalze 20 auf, die einen darauf befindlichen Streifen der Elastiklage 7, dargestellt in Fig. 2, auf der Oberfläche 1 auch nach dem Abschneiden festhält. Die Vakuumwalze 20 hat eine Umfangsgeschwindigkeit, die höher ist als die Zuführgeschwindigkeit des Materialstreifens der Elastiklage 7 von der Vorratswalze 15, dadurch wird der Distanzierungseffekt für die einzelnen Streifen der Elastiklage 7 erreicht.

In Fig. 3 ist angedeutet, wie gerade ein Streifen der Elastiklage 7 von der Vakuumwalze 20 der Schneide- und Distanzstation 17 auf die kontinuierlich durchlaufende Nichtelastiklage 8 aufgebracht wird, dort ungefähr beim Verlassen der Fältelungswalze 11, nämlich beim tangentialen Ablaufen von der Fältelungswalze 11, abgezogen durch die Abzugswalze 10. In Fig. 2 erkennt man einen schon vorher aufgebrachten Streifen der Elastiklage 7.

Durch die Darstellung der Breite des mit der Elastiklage 7 versehenen durchlaufenden Materialstreifens nicht elastischen Materials ist in Fig. 2 im übrigen angedeutet, daß dort das Herstellungsverfahren mit anschließender Querdehnung realisiert wird. Darauf kommt es aber im Zusammenhang mit der Lehre der vorliegenden Erfindung nicht an, so daß hierzu weitere Erläuterungen unterbleiben.

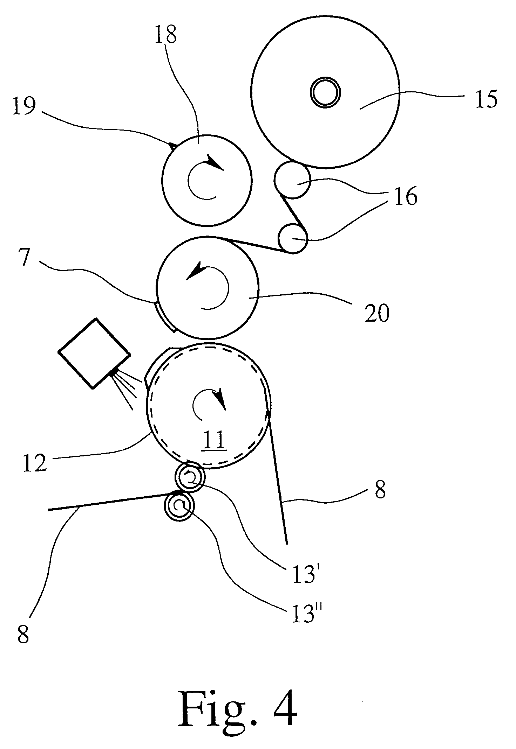

Das in Fig. 4 dargestellte weitere Ausführungsbeispiel einer Herstellungsvorrichtung arbeitet nicht mit mehreren in Umfangsrichtung hintereinander angeordneten Gruppen von Druckrollen 13, sondern mit zwei der Fältelungswalze 11 vorgeordneten Druckwalzen 13', 13'', die jeweils eine Gruppe von Druckrollen 13 zusammenfassen. Um diese weiteren Druckwalzen 13', 13'' wird die Nichtelastiklage 8 ebenfalls unter Zug geführt. Man erkennt, daß die Druckwalze 13' mit den Ringnuten 12 auf der Fältelungswalze 11 in Eingriff steht, während die Druckwalze 13'' mit den auf der Druckwalze 13' ihrerseits gebildeten Ringnuten 12 in Eingriff steht. Vorzugsweise ist auch hier ein Fortschreiten des Eingriffs von innen nach außen realisiert. Im übrigen stimmt die Vorrichtung aus Fig. 4 mit der Vorrichtung aus Fig. 2 und 3 überein.

## Patentansprüche

1. Verfahren zur Herstellung von Windeln od. dgl. mit mindestens einem dehnungselastischen Bereich (4), vorzugsweise angeordnet im Bundbereich,
wobei in diesen, Bereich (4) der Windel ein in Längsrichtung dehnbares, vorzugsweise streifenförmiges Dehnungselement (6) aus einer in Längsrichtung des Dehnungselementes (6) dehnbaren Elastiklage (7) und mindestens einer damit flächig verbundenen, insbesondere verklebten oder verschweißten Nichtelastiklage (8) angeordnet ist, dessen Nichtelastiklage (8) bei nicht gedehnter Elastiklage (7) gefältelt bzw. gekräuselt und bei gedehnter Elastiklage (7) gestrafft bzw. glatt ist,
bei dem die Nichtelastiklage (8) mit in Querrichtung (quer zur Längsrichtung des Dehnungselements (6)) verlaufenden Falten bzw. Kräuselungen versehen und in diesem Zustand stabilisiert wird,
bei dem die gefältelte bzw. gekräuselte Nichtelastiklage (8) mit der Elastiklage (7) verbunden, insbesondere verklebt oder verschweißt wird,
bei dem die in Längsrichtung dehnbare Elastiklage (7) bei dem zuvor genannten Verfahrensschritt nicht gedehnt ist und
bei dem das so hergestellte Dehnungselement (6) den weiteren Herstellungsschritten zugeführt wird,
**dadurch gekennzeichnet,**
daß die Herstellungs-Bewegungsrichtung die Querrichtung bezüglich des Dehnungselements (6) ist.

2. Verfahren nach dem vorhergehenden Anspruch, dadurch gekennzeichnet, daß die Elastiklage (7) aus flächigem Kunststoff, ggf. aus PU-Schaumstoff, insbesondere aber aus fluted elastic material besteht und/oder daß die Nichtelastiklage (8) aus Polyethelenfolie besteht und/oder daß zwei Nichtelastiklagen (8) mit der Oberseite und der Unterseite der Elastiklage (7), und zwar gleichzeitig oder nacheinander, verbunden werden.

3. Verfahren nach einem der vorhergehenden Ansprüche, dadurch gekennzeichnet, daß die Nichtelastiklage (8) während des Verbindens mit der Elastiklage (7) in der Herstellungs-Bewegungsrichtung kontinuierlich bewegt wird.

4. Verfahren nach einem der vorhergehenden Ansprüche, dadurch gekennzeichnet, daß die glatte Nichtelastiklage (8) von einer Vorratswalze durch eine Fältelungsstation zu einer Verbindungsstation mit der Elastiklage (7) gezogen und in der Fältelungsstation mit den in Bewegungsrichtung verlaufenden Falten versehen wird und, vorzugsweise, daß die Nichtelastiklage (8) in der Fältelungsstation unter Zug um eine Fältelungswalze (11) geführt wird, die über ihre Breite verteilt eine Mehrzahl von Ringnuten (12) aufweist, in die das Material der Nichtelastiklage (8) durch entsprechend eingreifende Druckrollen (13) faltenartig hineingedrückt wird.

5. Verfahren nach einem der vorhergehenden Ansprüche, dadurch gekennzeichnet, daß die Faltenbildung von innen nach außen fortschreitend erfolgt.

6. Verfahren nach einem der vorhergehenden Ansprüche, dadurch gekennzeichnet, daß die Nichtelastiklage (8) auf der Fältelungswalze (11) mehrere, insbesondere vier, in Umfangsrichtung hintereinander angeordnete Gruppen von Druckrollen (13) durchläuft.

7. Verfahren nach einem der vorhergehenden Ansprüche, dadurch gekennzeichnet, daß die der Fältelungswalze (11) zugeordneten Druckrollen (13) in einer Druckwalze (13') zusammengefaßt sind, um die die Nichtelastiklage (8) unter Zug herumgeführt wird und/oder daß eine weitere Gruppe von Druckrollen (13) in einer weiteren Druckwalze (13'') zusammengefaßt sind, um die die Nichtelastiklage (8) unter Zug geführt wird und die mit der ersten Druckwalze (13') in Eingriff steht.

8. Verfahren nach einem der vorhergehenden Ansprüche, dadurch gekennzeichnet, daß die Nichtelastiklage (8) nach Durchlaufen der Druckrollen (13) eine Klebemittel-Auftragstation (14) durchläuft und ungefähr beim Verlassen der Fältelungswalze (11), also beim tangentialen Ablaufen von der Fältelungswalze (11), mit der nicht gedehnten Elastiklage (7) verbunden wird und/oder daß die Nichtelastiklage (8) anstelle des Durchlaufens einer Klebemittel-Auftragstation (14) beim Verbinden mit der Elastiklage (7) - durch Ultraschallschweißung - verschweißt wird.

9. Verfahren nach einem der vorhergehenden Ansprüche, dadurch gekennzeichnet, daß die Elastiklage (7) von einer Vorratswalze (15) kontinuierlich abgezogen und zur Verbindungsstation geführt und kontinuierlich durchlaufend mit der Nichtelastiklage (8) verbunden wird und daß nachträglich ein Schneiden dieses Verbundes zu einzelnen streifenartigen Dehnungselementen (6) erfolgt.

10. Verfahren nach einem der vorhergehenden Ansprüche, dadurch gekennzeichnet, daß die Elastiklage (7) von einer Vorratswalze (15) kontinuierlich abgezogen, in einer Schneide- und Distanzstation (17) (cut and slip station) in Streifen geschnitten wird, und die Streifen in Herstellungs-Bewegungsrichtung auf Distanz gebracht werden und daß die so vereinzelten Streifen der Elastiklage (7) dann in der Verbindungsstation mit dem kontinuierlich durchlaufenden, gefältelten Material der Nichtelastiklage (8) verbunden werden.

11. Vorrichtung zur Herstellung von Windeln od. dgl. mit mindestens einem dehnungselastischen Bereich (4), vorzugsweise angeordnet im Bundbereich, wobei in diesem Bereich (4) ein in Längsrichtung dehnbares, vorzugsweise streifenförmiges Dehnungselement (6) aus einer in Längsrichtung des Dehnungselementes (6) dehnbaren Elastiklage (7) und mindestens einer damit flächig verbundenen, insbesondere verklebten oder verschweißten Nichtelastiklage (8) angeordnet ist, dessen Nichtelastiklage (8) bei nicht gedehnter Elastiklage (7) gefältelt bzw. gekräuselt und bei gedehnter Elastiklage (7) gestrafft bzw. glatt ist, insbesondere zur Durchführung eines Verfahrens nach einem der Ansprüche 1 bis 10,
**gekennzeichnet durch,**
eine Fältelungswalze (11), die über ihre Breite verteilt eine Mehrzahl von Ringnuten (12) aufweist,
mehrere, vorzugsweise vier, in Umfangsrichtung der Fältelungswalze (11) hintereinander angeordnete Gruppen von Druckrollen(13), deren jede in eine zugeordnete Ringnut (12) eingreift,
ggf. eine in Umfangsrichtung hinter der letzten Gruppe von Druckrollen (13) angeordnete Klebemittel-Auftragstation (14) oder durch eine Ultraschall-Schweißstation und eine das um die Fältelungswalze (11) kontinuierlich laufende Material der Nichtelastiklage (8) unter Zugspannung haltende Abzugswalze (10) od. dgl.,
wobei in Drehrichtung der Fältelungswalze (11) gesehen die erste Gruppe von Druckrollen (13) nur mit den in der Mitte der Fältelungswalze (11) liegenden Ringnuten (12) in Eingriff stehen und die dann folgenden Gruppen von Druckrollen (13) jeweils weitere äußere Ringnuten (12), insbesondere zusätzlich, erfassen.

12. Vorrichtung zur Herstellung von Windeln od. dgl. mit mindestens einem dehnungselastischen Bereich (4), vorzugsweise angeordnet im Bundbereich, wobei in diesem Bereich (4) ein in Längsrichtung dehnbares, vorzugsweise streifenförmiges Dehnungselement (6) aus einer in Längsrichtung des Dehnungselementes (6) dehnbaren Elastiklage (7) und mindestens einer damit flächig verbundenen, insbesondere verklebten oder verschweißten Nichtelastiklage (8) angeordnet ist, dessen Nichtelastiklage (8) bei nicht gedehnter Elastiklage (7) gefältelt bzw. gekräuselt und bei gedehnter Elastiklage (7) gestrafft bzw. glatt ist, insbesondere zur Durchführung eines Verfahrens nach einem der Ansprüche 1 bis 10,
**gekennzeichnet durch,**
eine Fältelungswalze (11), die über die Breite verteilt eine Mehrzahl von Ringnuten (12) aufweist,
eine mehrere Druckrollen (13), deren jede in eine zugeordnete Ringnut (12) eingreift, zusammenfassende Druckwalze (13') um die die Nichtelastiklage (8) unter Zug geführt wird,
ggf. eine weitere mehrere Druckrollen (13) zusammenfassende Druckwalze (13''), wobei diese Druckwalze (13'') mit der Druckwalze (13') in Eingriff steht und die Nichtelastiklage (8) unter Zug um diese Druckwalze (13'') geführt wird,
ggf. eine in Umfangsrichtung an der Fältelungswalze (11) hinter der letzten Gruppe von Druckwalze (13') angeordnete Klebemittel-Auftragstation (14) oder durch eine Ultraschall-Schweißstation und
eine das um die Fältelungswalze (11) und die Druckwalze (13' bzw. Druckwalzen 13' , 13'') kontinuierlich laufende Material der Nichtelastiklage (8) unter Zugspannung haltende Abzugswalze (10) od. dgl.,
wobei die Anzahl der Ringnuten (12), die die Druckwalze (13') für die Druckwalze (13'') bildet vorzugsweise geringer ist als die Anzahl der Ringnuten (12) auf der Fältelungswalze (11).
